# EUROPEAN PATENT SPECIFICATION

(11) **EP 4 017 449 B1**
(45) Date of publication and mention of the grant of the patent: **20.11.2024**
(21) Application number: 20760414.1
(22) Date of filing: 18.08.2020
(51) Int. Cl.: A61F 13/01, A61F 13/0206, A61F 13/0203, A61F 13/05, A61F 13/534, A61F 13/53, A61F 13/00, A61F 13/02

(54) **ABSORBENT COMPONENT**
ABSORBIERENDE KOMPONENTE
COMPOSANT ABSORBANT

(30) Priority: 22.08.2019 GB 201912076
(43) Date of publication of application: 29.06.2022
(73) Proprietor: T.J.Smith and Nephew,Limited, Hull HU3 2BN (GB)
(72) Inventor: WHELDRAKE, Amy Nicole, Hull HU3 2BN (GB)
(74) Representative: Smith & Nephew
(86) International application number: PCT/EP2020/073060
(87) International publication number: WO 2021/032713

(56) References cited:
- EP-A2- 0 342 927
- EP-A2- 0 342 927
- US-A1- 2017 065 458
- US-A1- 2017 065 458
- US-A1- 2019 021 912
- US-A1- 2019 021 912

## Description

### FIELD

The present disclosure relate to absorbent components useful in apparatuses, systems, and methods for the treatment of wounds, for example in wound dressings in combination with negative pressure wound therapy, or non-negative pressure wound therapy. The absorbent components, apparatuses, systems and methods can incorporate or implement any combination of the features described below.

### BACKGROUND

Many different types of wound dressings are known for aiding in the healing process of a human or animal. These different types of wound dressings include many different types of materials and layers, for example, gauze, pads, foam pads or multi-layer wound dressings. Topical negative pressure therapy, sometimes referred to as vacuum assisted closure, negative pressure wound therapy, or reduced pressure wound therapy, is widely recognized as a beneficial mechanism for improving the healing rate of a wound. Such therapy is applicable to a broad range of wounds such as incisional wounds, open wounds and abdominal wounds or the like.

However, in prior art dressings for use in wound therapy, the absorbent component of the wound dressing is often the most limiting in terms of its extension-recovery (stretchability) properties. The absorbent component is conventionally the component of the dressing which provides the greatest absorbency capacity but the lowest level of stretchability. The high absorbency of an absorbent component is often achieved through the use of superabsorbent particles (SAP) or superabsorbent fibre (SAF). However, the ways in which SAP or SAF are conventionally incorporated prevents them from possessing stretch properties. Therefore, when increased extensibility recovery is required in a wound dressing, the dressings have typically had thinner or smaller absorbent components with the disadvantage of reduced levels of absorbency.

EP 0 342 927 A2 and US2019/0021912 A1 disclose stitch-bonded nonwoven absorbent substrates.

### SUMMARY

Embodiments of the present invention are directed to an absorbent component and dressings comprising the absorbent components, as well as methods of treatment for wounds using the absorbent components and dressings according to the invention.

According to a first embodiment of the invention there is provided an absorbent component for a wound dressing comprising a stitch bonded nonwoven substrate wherein the nonwoven substrate is stitch bonded with an elastomeric yarn and wherein the nonwoven substrate comprises a a blend of superabsorbent material and/or a gelling material and non-superabsorbent and non-gelling support material(s).

Elastomeric materials are materials that possess high elongations (10%-800%) prior to breaking and that recover fully and rapidly from high elongations up to their breaking point. The elastomeric material may comprise any suitable elastomeric material. Suitable elastomeric materials include crosslinked natural and synthetic rubbers, spandex (segmented polyurethanes), and anidex (crosslinked polyacrylates). The elastomeric material may be any suitable material. Preferably the elastomeric material comprises spandex.

Because the yarn is elastomeric, the absorbent component has an inherent stretch and by virtue of the incorporation of a superabsorbent material and/or a gelling material, the absorbent component is also highly absorbent. Thus an absorbent component of a wound dressing may be produced that is both stretchable and has high absorbency properties.

The yarn may be any suitable type of yarn, for example the yarn may be a monofilament yarn, a spun yarn or a core spun yarn. When the yarn is a continuous filament yarn, the risk of fibres being left behind upon removal of a dressing containing such a yarn from a wound is significantly reduced. Where the yarn comprises more than one component, each of the components may be elastomeric or one or more of the components may be elastomeric.

The yarn may comprise at least 50% elastomeric material. More preferably the yarn may comprise at least 60%, at least 70%, at least 80% or at least 90% elastomeric material. In certain embodiments the yarn may consist essentially of an elastomeric material.

The elastomeric yarn may be a core spun yarn comprising a yarn core fibre and a secondary fibre disposed exteriorly around the yarn core fibre wherein the yarn core fibre comprises an elastomeric material. The secondary fibre disposed exteriorly around the yarn core fibre may comprise a superabsorbent material and/or a gelling material.

A nonwoven substrate is a sheet of fibres, continuous filaments, or chopped yarns of any nature or origin, that have been formed into a web by any means. The fibres, continuous filaments or chopped yarns are herein after referred to collectively as fibres.

The nonwoven material may have any suitable weight and the most suitable weight may vary depending on the intended end use of the nonwoven material.

The nonwoven material may be produced by any suitable means, for example the nonwoven material may be wetlaid, for example wetlaid cellulose (paper), airlaid, carded, spun laid, meltblown or a combination of the above techniques. Preferably the nonwoven material is a carded nonwoven material.

The components of the nonwoven material are bonded together by stitch bonding after they have been laid to form and/or strengthen the nonwoven material. Stitch bonding is the process by which the fibres are connected by stitches sewn or knitted through the web.

The web may be stitch bonded by any suitable means. Stitchbonding of the web can be carried out on known stitchbonding equipment. Particularly preferred equipment is a Maliwatt stitch bonding machine. For example, the web may be stitch bonded with a weaving machine via longitudinal yarns or by warp knitting. The nature of the absorbent component including its stretchability and surface nature may be controlled by the nature of the stitching pattern, stitching length, stitch gauge and stitch tension used when stitch bonding the nonwoven substrate. Preferred stitch gauges are 5-12 stitches per inch and preferred stitching patterns are running or zig-zag. The preferred tension of the elastomeric yarn is at a tautness which maintains the stitch and permits the desired stretch properties.

The stitch bonding may be the only means by which the nonwoven web is bonded together.

Alternatively the fibres of the nonwoven material may be additionally bonded either before or after the stitch bonding by a further suitable means, for example by chemical, thermal or mechanical means. For example the nonwoven material may comprise a thermoplastic component and the nonwoven material may be bonded by the application of heat to the thermoplastic component of the material. Alternatively the nonwoven material may be bonded by applying a binder to the web by impregnating, spraying or coating the web with a binder. The binder may comprise a polymeric binder such as an acrylate polymer or its copolymers. The fibres of the nonwoven material may alternatively or additionally be needled together.

The superabsorbent material may comprise any suitable superabsorbent material.

A superabsorbent material is typically capable of absorbing many times its own mass of water, for example up to 200, 300 or more times its own mass of water. A gelling material is capable of absorbing aqueous fluid and on absorbing said fluid becomes gel-like, moist and slippery. The absorbent material may be both a gelling material and a superabsorbent material.

Examples of suitable superabsorbent materials include a polysaccharide or modified polysaccharide, a polyvinylpyrrolidone, a polyvinyl alcohol, a polyvinyl ether, a polyurethane, a polyacrylate, a polyacrylamide, collagen, a cellulose, gelatin, or mixtures thereof.

The gelling material may comprise any suitable gelling material.

Examples of suitable gelling materials include polysaccharides or modified polysaccharides, for example pectins, alginates, chitosans, hyaluronic acid and celluloses.

The gelling material may comprise a pectin or an alginate.

The superabsorbent material may comprise a polysaccharide or modified polysaccharide. Suitable polysaccharides include pectins, chitosans, alginates, and mixtures of alginate and other polysaccharides.

The superabsorbent material may comprise a cellulose. Preferably the superabsorbent material comprises a hydrophilically modified cellulosic material such as methyl cellulose, carboxymethyl cellulose (CMC), carboxyethyl cellulose (CEC), ethyl cellulose, propyl cellulose, hydroxyethyl cellulose, hydroxypropyl cellulose, hydroxypropylmethyl cellulose, carboxyethyl sulfonate cellulose, cellulose alkyl sulfonate, or mixtures thereof.

The cellulose may comprise a carboxymethyl cellulosic material, or cellulose alkyl sulfonate.

The cellulose may comprise a carboxymethyl cellulosic material.

The cellulose may comprise cellulose alkyl sulfonate. The alkyl moiety of the alkyl sulfonate substituent group may have an alkyl group having 1 to 6 carbon atoms, such as methyl, ethyl, propyl, or butyl. The alkyl moiety may be branched or unbranched, and hence suitable propyl sulfonate substituents may comprise 1- or 2-methyl-ethylsulfonate. Butyl sulfonate substituents may comprise 2-ethyl-ethylsulfonate, 2,2-dimethyl-ethylsulfonate, or 1,2-dimethyl-ethylsulfonate. The alkyl sulfonate substituent group may comprise ethyl sulfonate. The cellulose alkyl sulfonate may be as described in WO10061225 or US2016/114074, or 2006/0142560 or US 5,703,225.

The hydrophilically modified cellulosic material may have varying degrees of substitution, the chain length of the cellulose backbone structure, and the structure of the alkyl sulfonate substituent. Solubility and absorbency are largely dependent on the degree of substitution: as the degree of substitution is increased, the cellulose alkyl sulfonate becomes increasingly soluble. It follows that, as solubility increases, absorbency increases. Such materials preferably have a degree of substitution of at least 0.2 carboxymethyl groups per glucose unit, or at least 0.3 or at least 0.5.

The superabsorbent material and/or gelling material may comprise a combination or blend of two or more different superabsorbent and/or gelling materials.

Preferably the superabsorbent material and/or gelling material is a superabsorbent fibre or a gelling fibre, most preferably a superabsorbent fibre.

Methods for producing superabsorbent materials and gelling materials and fibres thereof are known in the art, and any suitable method may be employed to produce materials or fibres for use in the present invention. Fibres for use in the invention may be further processed by any suitable method known in the art, including washing, crimping, carding, spinning and/or cutting.

The superabsorbent material and/or gelling material may be blended with additional starting materials for production of the nonwoven substrate, such as support materials, prior to the fabrication of the nonwoven substrate. The superabsorbent material and/or gelling material may be blended in any suitable way. For example the superabsorbent material and/or gelling material may be homogeneously blended with any additional starting materials or a gradient composition may be produced.

Alternatively a superabsorbent and/or gelling material may be impregnated into the nonwoven substrate after the nonwoven substrate has been fabricated.

Preferably the nonwoven substrate comprises at least 30%, at least 40%, at least 50%, at least 60%, at least 70%, at least 80%, or at least 90% superabsorbent and/or gelling material.

The non-superabsorbent and non-gelling support materials may be any suitable materials known in the art, or may be a mixture of two or more non-superabsorbent and non-gelling support materials. The non-superabsorbent and non-gelling support materials may comprise textile materials, and may comprise natural materials (e.g. cotton), natural materials which have been modified (e.g. cellulosic fibres such as viscose or lyocell (sold under the trade name TENCEL)), or synthetic (e.g. polyester, polypropylene or polyamide) materials. Different materials and fibres thereof have different characteristics in terms of tensile strength and absorbency, and appropriate non-superabsorbent and non-gelling support materials may be chosen according to the desired characteristics of the nonwoven substrate. In addition, a combination of two or more non-superabsorbent and non-gelling support materials may be used in order to achieve the desired characteristics. Preferably, the non-superabsorbent and non-gelling support materials are natural materials or fibres thereof which have been modified or synthetic materials. More preferably, the non-superabsorbent and non-gelling support materials are cellulosic materials or fibres thereof or polyester or polyamide materials or fibres thereof, most preferably polyester or polyamide materials or fibres thereof.

The use of non-superabsorbent and non-gelling support materials may aid in maintaining the integrity of the nonwoven substrate when wetted.

When the non-superabsorbent and non-gelling support materials are non-superabsorbent and non-gelling support fibres, the fibres may be crimped or otherwise textured. By crimping or texturizing the fibres, the processing of the blend of superabsorbent and/or gelling and non-superabsorbent and non-gelling support fibres may be facilitated as superabsorbent fibres and/or gelling fibres alone can be difficult to process.

The absorbent component may comprise more than one layer. The absorbent component may comprise a non-superabsorbent and non-gelling nonwoven layer. The non-superabsorbent and non-gelling nonwoven layer and the superabsorbent and/or gelling nonwoven layer may be stitch bonded together as a single component or may be separately stitch bonded. For example the nonwoven element comprising the superabsorbent and/or gelling material may be sandwiched between two non-superabsorbent and non-gelling nonwoven layers prior to stitch bonding all three layers with an elastomeric yarn.

Further disclosed herein is a method of manufacturing an absorbent component according to the first aspect of the invention. The method of manufacture is not part of the claimed nvention.

Said method of producing an absorbent component according to the first aspect of the invention comprises the steps of:
(i) providing a plurality of fibres, optionally wherein the plurality of fibres comprises superabsorbent and/or gelling fibres
(ii) laying a web of said fibres
(iii) incorporating a superabsorbent and/or gelling material
(iv) stitch bonding the web with an elastomeric yarn
Wherein step (iii) may alternatively be performed after step (iv) and wherein if step (i) comprises superabsorbent and/or gelling fibres, step (iii) is optional.

The superabsorbent and/or gelling material may be incorporated into the absorbent component by blending the superabsorbent and/or gelling fibres together with any supporting fibres in step (i).

The plurality of fibres in step (i) may consist of superabsorbent and/or gelling fibres.

The method may further comprise the step of bonding the fibres by a secondary means. Said secondary means may comprise chemical, thermal or mechanical means. For example the nonwoven material may comprise a thermoplastic component and the nonwoven material may be bonded by the application of heat to the thermoplastic component of the material. Alternatively the nonwoven material may be bonded by applying a binder to the web by impregnating, spraying or coating the web with a binder. The binder may comprise a polymeric binder such as an acrylate polymer or its copolymers.

The elastomeric yarn may have any of the features described above in relation to the first aspect of the invention. The absorbent component may incorporate other components useful in wound treatment. For example, medicaments, antimicrobial, antibacterial or antiseptic materials, or odour control materials. Suitable components are known to the skilled person as are materials which incorporate such components. Suitable components may be incorporated into the absorbent component in any one or more of steps (i) to (iv) or subsequently to production of the absorbent component.

The invention further provides a wound dressing which comprises or consists of the absorbent component of the invention. Thus according to a second aspect of the invention there is provided a wound dressing comprising an absorbent component according to the first aspect of the invention. Such a dressing may take the form of an absorbent component as set out above in relation to the first aspect of the invention. The wound dressing may comprise further components which are typical of absorbent wound dressings, for example, a vapour permeable, liquid impermeable backing layer or an adhesive wound contacting layer. Further typical features of negative pressure and non-negative pressure wound dressings are set out below.

Advantageously any further components of the wound dressing according to the invention may also be stretchable to a similar degree to the absorbent component according to the first aspect of the invention. This allows the wound dressing as a whole to stretch in use improving the comfort of the patient and the efficacy of the wound dressing.

The elastomeric absorbent component may also be suitable for use as a wound packing material. A wound packing material is a material used to fill a cavity wound and is of particular use in conjunction with negative pressure wound therapy applications where a wound packing may be used to prevent a wound dressing from entering a wound cavity. For such applications, the component may be produced with suitable dimensions.

The absorbent component according to the first aspect of the invention, or the wound dressing of the second aspect of the invention may be of use in a method of treating a wound. The method of treating a wound is not part of the invention claimed.

The method of treating a wound comprises placing a wound dressing according to the second aspect of the invention or an absorbent component according to the first aspect of the invention over a wound.

The method of treatment may be a conventional wound therapy treatment or a negative pressure wound therapy treatment.

Such a method may comprise the step of placing an absorbent component according to the first aspect of the invention, or a wound dressing comprising said absorbent component over a wound.

The treatment may be a conventional, i.e. non-negative pressure, or negative pressure wound therapy treatment.

According to a fourth aspect of the invention is provided a negative pressure apparatus comprising a wound contact layer, an absorbent component according to the first aspect of the invention, a transmission layer, a cover layer and an orifice to allow a negative pressure to be applied.

### BRIEF DESCRIPTION OF DRAWINGS

Embodiments of the present disclosure will now be described hereinafter, by way of example only, with reference to the accompanying drawings in which:
FIG. 1 illustrates a perspective view of an absorbent component according to the invention;
FIG. 2 illustrates a view through an embodiment of a wound dressing of the present disclosure;
FIG. 3A illustrates a plan view of the dressing of Figure 2;
FIG. 3B illustrates a perspective view of the dressing of Figure 2
FIG. 4A illustrates an alternative dressing arrangement;
FIG. 4B illustrates another dressing arrangement;
FIG. 5A illustrates an embodiment of a wound dressing;
FIG. 5B illustrates another embodiment of a wound dressing; and
FIG. 6 illustrates a top view of an embodiment of a wound dressing.

### DESCRIPTION OF EMBODIMENTS

Disclosed herein apparatuses and methods of treating a wound with or without reduced pressure, including for example a source of negative pressure and wound dressing components and apparatuses. The apparatuses and components comprising the wound overlay and packing materials or internal layers, if any, are sometimes collectively referred to herein as dressings. In some embodiments, the wound dressing can be provided to be utilized without reduced pressure.

The disclosed wound therapy is suitable for a human or animal body. Therefore, any reference to a wound herein can refer to a wound on a human or animal body, and any reference to a body herein can refer to a human or animal body.

The disclosed technology may relate to preventing or minimizing damage to physiological tissue or living tissue, or to the treatment of damaged tissue e.g., a wound as described above.

As used herein the expression "wound" may include any injury to living tissue and may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound becomes a chronic wound when it does not follow the healing stages resulting in a lengthened recovery. It is believed that the transition from acute to chronic wound can be due to a patient being immuno-compromised.

Chronic wounds may include for example: Venous ulcers: Venous ulcers usually occur in the legs, account for the majority of chronic wounds, and mostly affect the elderly, Diabetic ulcers (typically foot or ankle ulcers, Peripheral Arterial Disease, Pressure ulcers, or Epidermolysis Bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, bums, diabetic ulcers, pressure ulcers, stoma, surgical wounds, trauma and venous ulcers or the like.

The wound may also include a deep tissue injury. The deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

The wound may also include tissue at risk of becoming a wound as discussed above. For example, tissue at risk may include tissue over a bony protuberance (at risk of deep tissue injury/insult), pre-surgical tissue (e.g. knee) that may has the potential to be cut (for joint replacement/surgical alteration/reconstruction).

The disclosed technology relates to a method of treating a wound with the technology disclosed herein in conjunction with one or more of the following: advanced footwear, turning a patient, offloading examples such as diabetic foot ulcers, treatment of infection, systemix, antimicrobial, antibiotics, surgery, removal of tissue, affect blood flow, physiotherapy, exercise, bathing, nutrition, hydration, nerve stimulation, ultrasound, electrostimulation, oxygen therapy, microwave therapy, active agents ozone, antibiotics, antimicrobials, and the like.

The wound may be treated using topical negative pressure and/or traditional advanced wound care i.e., not aided by the using of applied negative pressure (may also be referred to as non-negative pressure therapy).

Advanced wound care may include use of an absorbent dressing, an occlusive dressing, use of an antimicrobial and/or debriding agents in a wound dressing or adjunct, a pad e.g., a cushioning or compressive therapy (such as stocking or bandages).

Treatment of such wounds can be performed using traditional wound care, wherein a dressing can be applied to the wound to facilitate and promote healing of the wound.

The wound dressings that may be utilized in conjunction with the disclosed technology include any known dressing in the art. The technology is applicable to negative pressure therapy treatment as well as non-negative pressure therapy treatment.

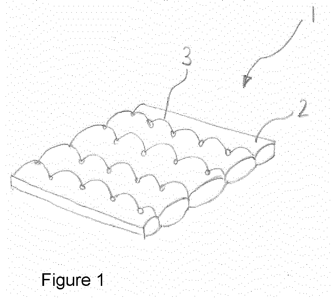

Referring first to Figure 1, there is shown an absorbent component generally designated 1 according to a first aspect of the invention. A stitch bonded nonwoven substrate 2 is provided. The nonwoven substrate is stitch bonded with an elastomeric yarn 3. The nonwoven substrate comprises a superabsorbent material and/or a gelling material in the form of a web of superabsorbent and/or gelling fibres.

The absorbent component 1 is stretchable due to the elastomeric properties of the stitch bonding elastomeric yarn 3.

The nonwoven substrate is generally produced by air laying the superabsorbent and/or gelling fibres although any suitable method may be used. The nonwoven substrate may further comprise thermoplastic fibres in which case the fibres may also be bonded by the application of heat in addition to being stitch bonded.

The absorbent component 1 may be incorporated into a wound dressing.

Typically a wound dressing according to the invention may comprise a further absorbent layer or layers in addition to the absorbent component according to the first aspect of the invention. The additional absorbent layer may be a knitted or woven material, a foam, a superabsorbent or a combination thereof.

The absorbent component 1 may also be incorporated into a negative pressure apparatus further comprising a wound contact layer, a transmission layer, a cover layer and an orifice for applying reduced pressure.

In order to improve the stretchability of the wound dressing according to the invention as a whole, the absorbent component according to the invention may be the only absorbent component in the wound dressing.

For the dressings according to the invention, the absorbent component may be contained between a wound contact layer and a top film.

The wound contact layer can comprise a perforated wound-side adhesive which can be a silicone adhesive, or a low-tack adhesive to minimise skin trauma on removal. The wound contact layer comprises a support material which can be a mesh, a net or a perforated film. It can also comprise a construction adhesive on the pad side, to ensure its intimate contact with the lowest part of the pad, and therefore efficient uptake of fluid from the wound without pooling.

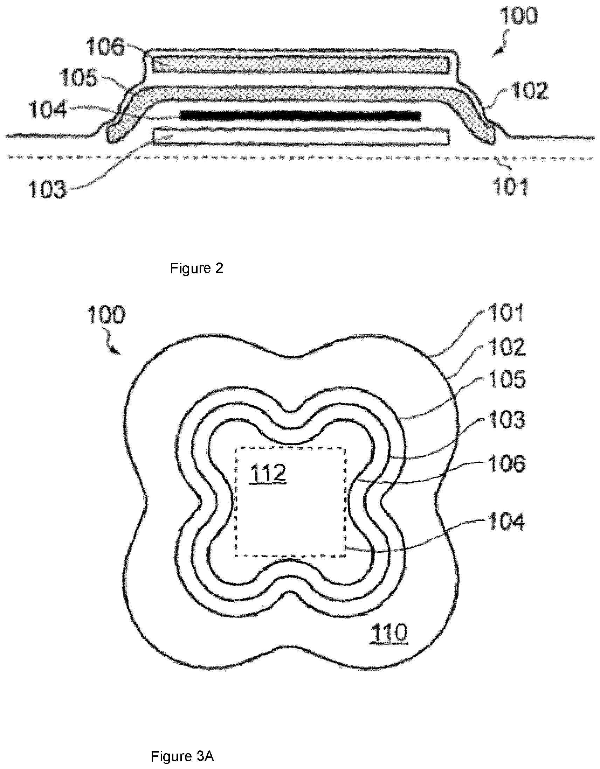

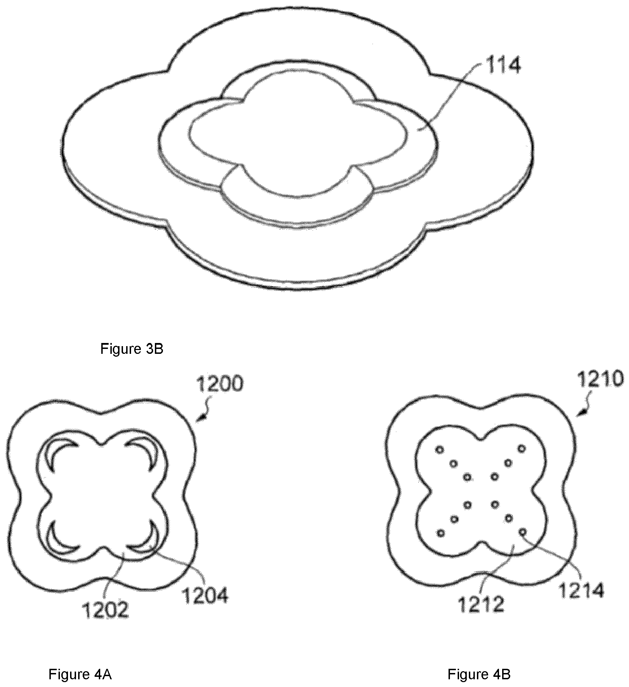

The top film is a liquid-impermeable, moisture-vapour permeable, breathable film, which allows moisture to evaporate from the dressing. Figures 2, 3A and 3B respectively show a schematic cross-sectional view, a plan view and a perspective view of a wound dressing according to an embodiment of the present disclosure. The wound dressing 100 includes a number of layers that are built up in a generally laminar fashion to form a dressing having a relatively planar form. The wound dressing 100 includes a border region 110 extending around the outer periphery of the dressing and a raised central region 112 in the centre of the dressing (in plan view). The precise dimensions of the border region and the central region may be predetermined to suit a particular wound or particular wound type. There may be no border region required. Here the border region has the general function of providing an area for sealingly engaging with a patient's skin surrounding a wound site to form a sealed cavity over the wound site. The central region is the location of further functional elements of the wound dressing.

The dressing 100 includes a perforated wound contact layer 101and a top film 102.

Further components of the wound dressing 100 include:
- A layer of polyurethane hydrocellular foam 103 of a suitable size to cover the recommended dimension of wounds corresponding to the particular dressing size chosen
- A layer of activated charcoal cloth 104 of similar or slightly smaller dimensions than 103, to allow for odour control with limited aesthetic impact on the wound side.
- A layer comprising the absorbent component of the invention 105, of dimensions slightly larger than 103 to allow for an overlap of superabsorbent material acting as leak prevention
- A layer of three-dimensional knitted spacer fabric 106, providing protection from pressure, while allowing partial masking of the top surface of the superabsorber, where coloured exudate would remain. In this embodiment this is of smaller dimension (in plan view) than the layer 105, to allow for visibility of the edge of the absorbent layer, which can be used by clinicians to assess whether the dressing needs to be changed.

In this embodiment the wound contact layer 101 is a perforated polyurethane film that is coated with a skin-compatible adhesive, such as pressure sensitive acrylic adhesive or silicone adhesive (not shown). Alternatively the wound contact layer may be formed from any suitable polymer, e.g. silicone, ethylvinyl acetate, polyethylene, polypropylene, or polyester, or a combination thereof. The skin-compatible adhesive is coated on the lower side of the layer 101, i.e. the side that is to contact the patient. Aptly the adhesive is coated as a continuous layer on the underside of the layer 101. Optionally the adhesive may be coated in a semi-continuous layer such as in a pattern such as a chequerboard pattern, polka dot pattern, herring bone pattern, mesh pattern or other suitable pattern. Alternatively the adhesive may be coated around a border region 110 of the dressing only, and not in a central region 112 of the dressing (as viewed from above in plan view) such that the adhesive may adhere to skin surrounding a wound and not the wound itself. The perforations allow the wound contact layer to be permeable to liquid and gas. The perforations are through holes extending from an upper surface to a lower surface of the wound contact layer to enable fluid to flow through the layer. The perforations are small enough to help prevent tissue ingrowth into the wound dressing yet still allow fluid to flow. The perforations may be slits or holes having a size range of 0.025 mm to 1.2 mm for example. The upper surface of layer 101 may optionally be coated with adhesive, to help in the construction of the dressing. Aptly the adhesive may be a pressure sensitive adhesive and aptly the same adhesive as used on the lower surface of the layer 101.

The layer 103 of polyurethane hydrocellular foam is located over the wound contact layer 101 and extends over the central region 112 of the wound contact layer.

The term hydrocellular is a term given to foams that are absorbent, hydrophilic and polymeric. The foams may have a particular range of cell size of 30 microns to 700 microns.

The foam is in this case of polyurethane, hydrophilic, conformable, resilient, and porous and allows fluids such as wound exudate to be drawn away from the wound site and further into the dressing. However, the foam also maintains a sufficiently moist wound healing environment so as to not dry out the wound, retaining a balanced moist atmosphere under the dressing. An optimal wound healing environment generally requires the area of the wound to have some level of moisture yet without excessive fluid.

The foam may be any suitable polymer foam. The foam is aptly a highly conformable hydrophilic foam, aptly an open celled foam, and more aptly the foam is a mixture of open and closed cells.

It is desirable that the foam layer absorbs the wound exudate rapidly. Such rapid absorption prevents undesirable pooling of exudate between the dressing and the wound.

The ability of polymer foam layers to absorb and retain fluids depends to some extent on the size of the foam cells, the porosity of the foam and the thickness of the foam layer. Suitable open cell foams of dressing embodiments of the present disclosure have a cell size of 30 microns to 700 microns and aptly a cell size of 50 microns to 500 microns. Apt open cell hydrophilic foams of dressings of the present disclosure have 20% to 70% and preferably 30% to 60% of the total membrane area of the cells as membrane openings. Such open cell foams permit transport of fluid and cellular debris into and within the foam.

Apt foams may be polyurethane, carboxylated butadiene styrene rubber, polyacrylate or the like foam. Such foams may be made of hydrophilic materials per se or may be treated to render them hydrophilic, for example with surfactants. It is preferred to use foams that are made of polymer that is itself hydrophilic as it has been found the exudate is less likely to coagulate rapidly. Favoured hydrophilic polymer foams are hydrophilic polyurethane and especially those which are made of crosslinked hydrophilic polyurethane. Preferred foams can be made by reacting a hydrophilic isocyanate terminated polyether prepolymer with water. Suitable hydrophilic polyurethane foams of this type include those known as HypolTM foams. HypolTM foams can be made from Hypol hydrophilic prepolymers marketed by W. R. Grace and Co and are hydrophilic cellular foams having a mixture of open and closed cells. HypolTM based foams are also available from Dow Chemicals. Other suitable foams are described in WO91/01706 in relation to the absorbent layer described, incorporated herein by reference, and in WO93/04101 also incorporated herein by reference.

The use of such foams of hydrophilic polymer in the absorbent pad of dressings of the present disclosure can allow the wound to be maintained in a moist condition even when the exudate produced has been absorbed and removed from the wound surface.

A further function of the foam layer is to wick away excess fluid from the wound area via its open cells. It is noted that PU foam itself can absorb liquid, the whole polymer swelling.

The odour-removing layer of activated charcoal cloth 104 is provided over the layer of foam 103. In this embodiment the activated charcoal layer is about the same length and depth as the foam layer and therefore lies over the foam layer to cover about the same area. The layer may be of Zorflex^{®} cloth available from Chemviron Carbon, for example. Alternative suitable materials are manufactured by MAST under the trade name C-TeX ^{®}.

The function of the odour-removing layer is to help prevent or reduce odour originating from the wound from transmitting out of the dressing.

It is noted that in this example the odour-removing layer is provided as a loose layer, unbonded to the adjacent layers, though alternatively the layers may be bonded by adhesive or stitching, etc.

The layer of absorbent material 105 is provided over the odour-removing layer 104. The absorbent layer 105 extends fully over the layer 104, as well as over the side portions of both the odour-removing layer 104 and foam layer 103.

The layer 105 forms a reservoir for fluid, particularly liquid, removed from the wound site and draws those fluids towards a cover layer 102. The material of the absorbent layer also prevents liquid collected in the wound dressing from flowing freely once in the dressing structure. The absorbent layer 105 also helps distribute fluid throughout the layer via a wicking action so that fluid is drawn from the wound site and stored throughout the absorbent layer, i.e. transferring and locking in the liquid. This prevents agglomeration in areas of the absorbent layer. The capacity of the absorbent material should be sufficient to manage the exudate flow rate of a wound for the predetermined life of the dressing, whether the wound is acute or chronic. Again, in combination with the foam layer, the layer 105 aptly should not cause the wound to become completely dry. This might occur if, for example, the superabsorbent material were to dry out the foam layer and then subsequently the wound area.

The absorbent layer 105 aptly has a high osmotic potential so as to prevent liquid being released from the layer, even when the layer is under compression (e.g. if the dressing area is pressed or leant on). Liquid may however leave the layer by diffusion through evaporation or possibly wicking transfer to a further layer.

The layer of absorbent material 105 may be of any suitable dimensions. Aptly, if the layer is shaped to a size larger than the layers between itself and the wound contact layer, then the layer can fold over the edges of any intermediate layers, acting as an enclosure such that any fluid moving into the dressing will encounter the absorbent layer prior to encountering the top film or the adhesive wound contact layer. This helps to prevent possible leaks of fluid from the dressing. As an alternative, a ring shaped (annular or torus) or other suitable border shaped portion of absorbent material may be added to a dressing separately from the absorbent layer to surround underlying layers and to perform the same function as the overlying edge of the absorbent layer.

An equilibrium is set up within the dressing core whereby moisture passes from the superabsorber to the top film and the fluid vapour starts to be transpired. A moisture gradient may be established within the dressing to continually remove fluid from the wound bed.

The shielding layer 106 is a layer having a 3-dimensional structure that may include open cell foam (e.g. AlleyvnTM foam by Smith & Nephew, Biatain foam by Coloplast or Advanced Medical Devices' ActivHeal foam), a knitted or woven spacer fabric (for example Baltex 7970 weft knitted polyester or Baltex XD spacer fabric or Surgical Mesh's Polyester felt or Polyester mesh) or a nonwoven fabric (e.g. Fiberweb's S-tex or Securon). Alternatively the shielding layer may be a completely opaque polymer film having cut-out windows or perforations, for example (e.g. SNEF's H514 or H518 blue net). Here the layer 106 is of polyester that includes a top layer (that is, a layer distal from the wound in use), which is a 84/144 textured polyester, a bottom layer (that is, a layer that lies proximate to the wound in use), which is a 100 denier flat polyester and a third layer formed sandwiched between these two layers, which is a region defined by a knitted polyester viscose, cellulose or the like monofilament fibre. Of course other materials and other linear mass densities of fibre could be used, including for example a multistrand alternative. The shielding layer 106 may be similar or identical to the materials described in US2011/0282309 in relation to the transmission layer.

The layer 106 allows the transmission therethrough of any gas or vapour to the top film 102 and may therefore be considered as a transmission layer.

Aptly the layer 106 performs one or more further functions including acting as a partial masking layer and acting as a force distributing (impact protection) layer.

Partial masking of wound exudate, blood or other matter released from a wound may be achieved with overlapping perforated fabrics disposed somewhat offset from each other. A shielding layer is itself formed from 3 sub-layers, such as the 3-D knit material described above. The perforated top and bottom layers allow transport of vapour and gas, and are offset from gas pathways in the central knitted layer. As such, vapour and gas may travel through the layer, but the coloured exudate cannot be seen, or travel, through the layer.

Alternatively a perforated cover layer 1102 is provided over a perforated shielding layer 1106, which allows moisture vapour and gas to be transmitted away from the dressing, yet provides sufficient masking for exudates to be visible only to a trained clinician.

More specifically, it is known that when a film is breathable (able to transmit vapour), then it is likely to allow colour to transmit therethrough. Even if a breathable film includes a coloured pigment for masking a lower layer, when exudate fluid contacts the film, coloured elements in the exudate can be carried into contact with the film and change the colour perception from the film, and be visible to the user. This allows fluid to transmit through the layer towards the top film whilst coloured solids or liquids remain bound in the absorbent layer below. Exudate colour is principally due to proteins and biological break down products from tissue or blood cells, which tend to be large molecules.

Another function of the shielding layer 106 may be for pressure distribution and impact protection. For example, if the patient accidentally knocks the wound area, leans on the wound area or another cause applies a pressure to the dressing covering a wound. Aptly the shielding layer is provided closer to where the pressure is being applied than other layers of the dressing.

The shielding layer 106 acts as a pressure spreading component, receiving a pressure on one side thereof (possibly a point force) and spreading the pressure over a wider area, thus reducing the relative pressure received on the other side of the shielding layer. As such, the level of pressure felt by the patient at the wound site is reduced.

A form of shielding layer that has been found to be a good pressure distributed is a layer having non-ordered fibres or strands, i.e. fibres lying at different angles with respect to each other, for example the knitted spacer fabric of Baltex 7970.

The absorbent layer 105 may also act as a pressure spreading component. A combination of the shielding layer 106 and the absorbent layer 105 has been found to give particularly apt pressure distributing properties. However, only one pressure spreading component may be sufficient.

In general, a material that is relatively non-deformable is more suitable for spreading point pressure. However this should be balanced by the requirement for deformation ability for the dressing to adhere to a non-planar body part.

When a pressure occurs from inside the patient's body, such as pressure from a protruding bone, the shielding layer may be somewhat less efficient at spreading the pressure if it is positioned towards the distal part of the dressing. However, any equal and opposite reaction of force acting back toward the patient's skin will be spread by the shielding layer 106 and absorbent layer 105 (e.g. if the patient is laying on something hard such as the ground or hard chair). The pressure spreading response will depend somewhat upon the hardness of the surface against which the patient and dressing are pressed against, if any.

The pressure spreading ability of these layers may also be useful against slower, constant pressures as well as rapid point forces.

The top film 102 is a cover layer for covering the lower layers of the dressing, helping to encapsulate the layers between the wound contact layer and the top film. The top film 102 is in this case a layer of polyurethane, Elastollan (trade name) SP9109 manufactured by BASF. The top film may be coated with any suitable adhesive. Aptly the adhesive will be a pressure sensitive adhesive e.g. acrylic adhesive or silicone adhesive.

As such, the top film 102 helps to ensure that the dressing remains breathable, i.e. allows a proportion of fluid absorbed in the dressing to be evaporated via the outer surface of the dressing. In this way certain fluid content of the exudate can be transpired from the dressing, reducing the volume of remaining exudate and increasing the time before the dressing becomes full. Also, the wound contact layer 101 and top cover 102 help to ensure that the border region 110 of the dressing remains breathable, i.e. allows a patient's normal skin perspiration to be evaporated through the dressing, which helps in preventing or minimising skin maceration.

The outer layer of dressings of the present disclosure when present can be a continuous conformable film. The continuous moisture vapour transmitting conformable film outer layer of the wound dressing may be used to regulate the moisture loss from the wound area under the dressing and also to act as a barrier to bacteria so that bacteria on the outside surface of the dressing cannot penetrate to the wound area. Suitable continuous conformable films will have a moisture vapour transmission rate of at least 300, aptly from 300 to 5000 grams preferably 500 to 2000 grams/square meter/24 hrs at 37.5 C at 100% to 10% relative humidity difference. Such moisture vapour transmission rate of the continuous film allows the wound under the dressing to heal under moist conditions without causing the skin surrounding the wound to macerate. To ensure the use of an adhesive on the top film 102 does not reduce the moisture vapour transmission rate, a hydrophilic water dispersible adhesive may be used e.g. hydrophilic acrylic adhesives. Although, other suitable adhesive may also be used. Aptly adhesive may also be spread across the surface of the film in the form of a pattern such that a portion of the area of the film does not contain adhesive. E.g., use of a polka dot pattern whereby adhesive is not present in the dot area and 5 to 95%, or aptly 10 to 80%, more aptly 30 to 70%, more aptly 40 to 70%, more aptly 40 to 60%, more aptly 40 to 50% of the area of film does not contain adhesive. It will be apparent to those skilled in the art that any suitable pattern of adhesive layer may be used to produce a top film 102 that is not fully coated with adhesive and thus maximises the moisture vapour transmission rate. Other suitable materials for the cover layer are described in WO91/01706 in relation to the conformable moisture vapour transmitting outer layer.

Additionally, the top film may act as a further barrier to any remaining odour from being transmitted out of the wound dressing, since the top film may include through holes that allow molecules of a predetermined maximum size to pass therethrough.

Figures 3A and 3B show a possible shape of a dressing, useful for enhanced compatibility with body movement, where each layer is shaped to reduce the incident angle of the pad edge, and to provide somewhat independently moving sub-sections of the dressing. The dressing border, including the wound contact layer 101 and the top film 102 can also comprise slits, provided to further enhance the conformability on application by allowing the borders to overlap if needed.

Reverting back to Figure 2, it can be seen that the cross-section of the dressing includes various layers stacked in a contiguous manner so as to form a generally laminate structure. Preferably the dressing is moisture vapour permeable. The layers shown in Figure 2 are of different widths and dimensions, though other arrangements are also possible.

In the border region 110, the top film 102 abuts with the wound contact layer 101. A moisture vapour transmitting adhesive layer is provided (not shown) in the border region 110 between the layers 101, 102 to bond the layers in that region. Suitable adhesives that are moisture vapour transmitting include various acrylate ester copolymer and polyvinyl ether pressure sensitive adhesives for example as described in UK patent number 1280631. Aptly the adhesives may be copolymers of an acrylate ester with acrylic acid for example as described in UK patent application number 2070631.

The dimensions of the components are arranged so as to minimise the angle of incidence of the dressing edge. This helps to reduce rubbing of the dressing against textiles and reduced snagging of the dressing against textile, by reducing the change in profile of the dressing throughout the thickness of the dressing.

In use, a wound dressing as described above would be applied to a wound site of a patient with the surface of the wound contact layer 101 facing the wound site. Any wound exudate, blood or other wound fluid would travel into the dressing via the wound contact layer and sequential layers above the wound contact layer. Fluid would permeate through the foam layer, the activated charcoal layer, and then reach the absorber layer at which point preferably the liquid would not go any further and be retained by the absorber layer. On the other hand, gas and moisture vapour would be able to permeate further via the shielding layer and/or top film.

The dressing shape has a rotational symmetry about its centre point (in plan view). In this example the dressing has 4 lobes. The shape of the central region 112 matches the shape of the border region 110 such that the width of the border region is approximately equal around the entire dressing. Aptly the border may be between about 12.5 mm and about 29 mm. More aptly the border is about 25 mm. Of course the border size will depend on the full dimensions of the dressing. Other numbers of lobes may be used such as 3, 5, 6, 7, 8, etc. The isotropic nature of the dressing shape gives the advantage that the user is not required to orientate the dressing in a specific manner before applying the dressing to a wound. The shape also enables the dressing to be adaptable to various parts of the body.

The dressing shape with 4 sub areas (lobes) aptly gives a maximum pad area with respect to the border area, yet has increased flexibility compared to a square dressing.

In addition, the dressing of the disclosure may be arranged to prevent shear stress between layers from causing damage to the dressing. This is because the layers are generally not adhered together, other than the top film 102 and wound contact layer 101 being adhered in the border region 110. Thus even if friction or other energy from shear movement occurs, the energy is dissipated by the layers prior to reaching the patient.

The wound facing surface of a wound dressing may be provided with a release coated protector (not shown in the figures), for example a silicon-coated paper. The protector covers the wound contacting side of the dressing prior to application to a patient, and can be peeled away at the time of use.

Various modifications to the detailed arrangements as described above are possible. For example, dressings according to the present disclosure do not require each of the specific layers as described above with respect to Figure 2. Dressings may include only one layer, or any combination of the layers described above. Alternatively or additionally, the materials of the layers described above may be combined into a single layer or sheet of material to perform the functions of each layer by a single layer.

As noted above, each of the layers described may be used to give one or more function to the wound dressing. As such, each of the layer materials may be used separately or in any combination such that each material provides the given function.

The wound contact layer described above is an optional layer. If used, a wound contact layer may be of any suitable material, such as polyethylene (or polyurethane as described above) or other suitable polymer, and may be perforated for example by a hot pin process, laser ablation process, ultrasound process or in some other way so as to be permeable to fluids.

Although the dressing described above has been described having a border region and a central region this need not be the case. The dressing may be provided without an adhesive layer for attachment to the skin of a patient. Rather, another means may be provided for locating the dressing at the correct position over a wound, such as adhesive tape or a tied bandage.

The relative widths of the various layers may be all the same or different to those as shown in the figures.

The dressing pad assembly may optionally be arranged with layers so that odour control is placed between two layers of different rates of absorptions. The odour control layer can be a charcoal cloth (knitted, woven, felt, nonwoven), or any other textile, foam, gel, net or mesh impregnated with odour-control materials. Such odour control materials can be cyclodextrins, zeolites, ion-exchange resins, oxidising agents, activated charcoal powder. It is also possible to use said odour-control materials dispersed in any layer of the pad assembly, and not as a discrete layer.

The dressing may optionally include a means of partially obscuring the top surface. This could also be achieved using a textile (knitted, woven, or nonwoven) layer without openings, provided it still enables fluid evaporation from the absorbent structure. It could also be achieved by printing a masking pattern on the top film, or on the top surface of the uppermost pad component, using an appropriate ink or coloured pad component (yarn, thread, coating) respectively. Another way of achieving this would be to have a completely opaque top surface, which could be temporarily opened by the clinician for inspection of the dressing state (for example through a window), and closed again without compromising the environment of the wound.

The dressing may optionally be arranged such that it has enhanced compatibility with body movement. This could also be achieved using a different shape for the sub-areas, such as diamonds, triangles, or a plurality of such shapes tessellated across the area of the dressing. Alternatively, preferential folding lines may be scored within the thickness of the dressing material, and thus define independent sub-areas for adapting to movement. Alternatively, the layers could be bonded using an elastic material, such as a viscoelastic adhesive, which would allow shear between the layers but refrain them from becoming separated and shifting across the pad.

A dressing assembly may optionally be arranged where the flowing properties are being provided by given material layers, and where the respective position of these layers provides additional properties on top of those from the individual layers. Alternative arrangement of layers than that described above may still provide some of the properties sought.

For example, placing the shielding layer 106 below the absorbent layer 105 would still allow protection from point pressure, but would lose the masking ability of this layer, and would probably affect the transmission of fluid between the foam layer 103 and the absorbent layer 105.

Another example is the placement of the odour control layer or component further away from the wound: this can be seen as beneficial because some types of odour control work differently depending on whether they are wet or dry. Placing a colour-less odour control component towards the top of the dressing (anywhere above 105) could provide odour control properties without the visual impact that a black layer of charcoal cloth would have.

In another embodiment, the shielding layer 106 is of the same dimensions as 105, and clinical judgment of the exudate spread can be made by observing the spread of exudate through the masking layer. This embodiment has the advantage of completely masking unsightly exudate from the absorbent layer.

Alternatively or additionally, the shielding layer can be provided with full masking capability, and windows provided at discrete points of the layer for enabling judgement of the exudate spread below such layer. Examples of such windows are illustrated in Figures 4A and 4B. The dressing 1200 shown in Figure 4A includes a masking layer 1202 and crescent-shaped windows 1204 provided in the masking layer to extend through the layer allowing visibility of the dressing therebelow. The dressing 1210 of Figure 4B includes a masking layer 1212 and a number of holes 1214 therethrough acting as windows for viewing the state of the dressing therebelow. With the dressings 1200,1210,1220 the progress of exudate spread over the dressing and towards the edge of the dressing can be monitored. In other alternatives instructions may be given to change the dressing when the exudate reaches a predetermined distance from the edge of the dressing, such as 5 mm from the dressing edge or 7 mm from the dressing edge, etc. Alternatively a 'traffic light' system may be implemented whereby an electronic indicator shows green, amber or red light to indicate the spread of exudate in the dressing. Alternatively or additionally, another suitable indicator may be used for indicating the spread of exudate over the dressing.

In another embodiment, odour control is not provided by a separate layer (i.e. no layer 104), but instead the odour-control material (activated charcoal, cyclodextrin, ion exchange resin, or other) is dispersed throughout another layer. This can be envisaged within the foam 103, the superabsorbent structure 105, or as a coating onto the masking layer 106.

In addition or alternatively, the obscuring layer may be coated with or formed from a material with size-exclusion properties to help with masking the exudate from view. For example, such a layer could have its lowermost side (the side closer to the wound) coated with materials such as zeolites or clays such as bentonite or sepiolite (the charged surface of which will tend to attract proteins and protein derivatives containing chromophores), other inorganic powders or molecular sieves (e.g. amberlite), proteins (albumin, haemoglobin components with molecular weight 15 to 70 KDa), ionic complexes such as hemes (molecular weight 600 to 850 g/mol), which have the function of immobilising species above a certain size or molecular weight. For example, species having molecular weight above 100 g/mol.

The shielding layer may be coated with or be formed of a hydrophilic compound (e.g. polyesters, polyurethanes, polyureas, polysaccharides, etc.) for assisting in wicking moisture towards the surface of the dressing, helping breathability of the dressing.

The shielding layer may be combined with a cover layer, such as an opaque or dark pigmented top layer.

The shielding layer (acting as a masking layer) may be combined with an absorbent layer, for example by providing an absorbent layer that has been dyed, for example with a dark blue pigment to the fibres of a nonwoven or airlaid material.

The shielding layer (acting as a pressure relieving layer) may be combined with an absorbent layer. For example a fibrous superabsorber layer may be provided with a high density of fibres for spreading point pressure. Alternatively a hydrophilic foam may be moulded around pressure-redistributing structures of pillars or arrays of an elastomer material, for example.

Odour control can be combined with absorbency by dispersing particles of activated charcoal or other odour-catching material in the absorbent component of the invention.

The layers described herein may each be provided directly adjacent another layer or with further layers therebetween.

A wound dressing may be formed by bringing together the required layers. The method may include bringing layers together with adhesive over part or all of a layer. The method may be a lamination process.

Alternatively a wound dressing may be formed by bringing together layers as described with respect to Figure 2, in a contiguous laminar stack, and adhering the top film to the wound contact layer in a border region.

The methods above may include bringing layers together with adhesive over part or all of a layer. The method may be a lamination process.

Alternatively a wound dressing may be formed by bringing together layers as described with respect to Figure 2, in a contiguous laminar stack, and adhering the top film to the wound contact layer in a border region.

The present disclosure comprising a shaped pad and dressing border, working as more independent sub-units of the dressing than what can be seen for a standard square shape, yields better conformability with movement than standard shapes. The dressing remains conformable with the skin and comfortable to wear, allowing the patient to move whilst wearing the dressing, and without creating detrimental traction on the peri-wound skin, which could lead to slowing of wound healing.

Some embodiments of the present disclosure also help to reduce the unsightly appearance of a dressing during use, by using materials that impart partial masking of the dressing surface. The masking should preferably only be partial, to allow clinicians to access the information they require by observing the spread of exudate across the dressing surface. This property, which is very important in helping patients live better with their treatment, had not been achieved until now for absorbent, breathable dressings. The partial masking nature of the obscuring layer enables a skilled clinician to perceive a different colour caused by exudate, blood, by-products etc. in the dressing allowing for a visual assessment and monitoring of the extent of spread across the dressing. However, since the change in colour of the dressing from its clean state to a state with exudate contained is only a slight change, the patient is unlikely to notice any aesthetic difference. Reducing or eliminating a visual indicator of wound exudate from a patient is likely to have a positive effect on their health, reducing stress for example.

When the layer of absorbent material folds over the edges of any other lower layers, the absorbent layer helps to prevent fluid from being squeezed from the dressing at the dressing edge region, thereby causing leakage. Various known dressings previously suffered from the risk of delamination of layers caused by fluid being squeezed towards the edge of the dressing, being driven between the layers and possibly escaping at the edge of the dressing. This may occur for example in a border region where a wound contact layer meets a cover layer, and any intermediate layers of the dressing are adjacent that border region. Aptly the absorbent layer is useful in preventing the release of any liquid, especially in the direction of the border region or edge of the dressing.

Any of the dressing embodiments disclosed herein can be used in with a source of negative pressure, such as a pump. Any of the dressing embodiments disclosed herein can also be used with a pump and a fluid or waste collection canister that can be put in fluid communication with the pump and the dressing so that the pump draws fluid or waste from the wound into the collection canister.

Additionally, in any embodiments, the pump can be a piezoelectric pump, a diaphragm pump, a voice coil actuated pump, a constant tension spring actuated pump, a manually actuated or operated pump, a battery powered pump, a DC or AC motor actuated pump, a combination of any of the foregoing, or any other suitable pump.

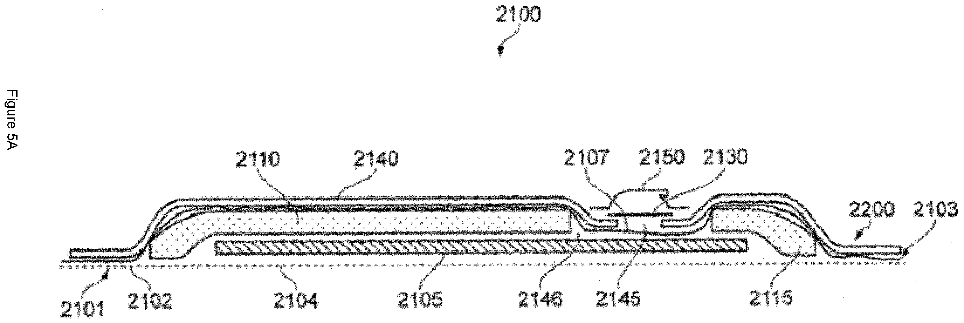

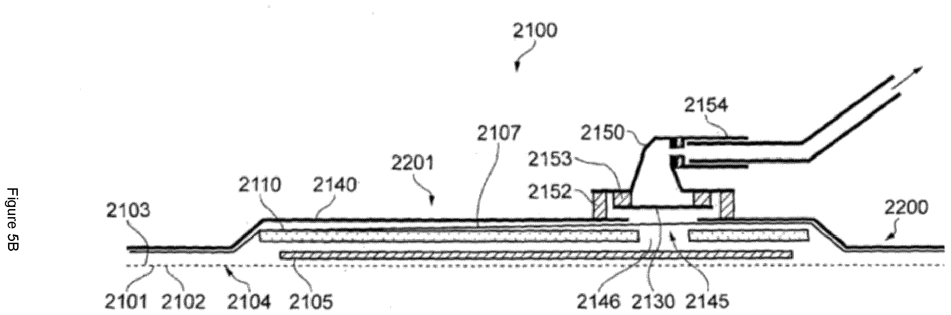

Figures 5A-B illustrate cross sections through a wound dressing 2100 according to an embodiment of the disclosure. A plan view from above the wound dressing 2100 is illustrated in Figure 16 with the line A-A indicating the location of the cross section shown in Figures 5A and 5B. It will be understood that Figures 5A-B illustrate a generalized schematic view of an apparatus 2100. It will be understood that embodiments of the present disclosure are generally applicable to use in TNP therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

According to a further aspect of the invention is provided a negative pressure apparatus comprising a wound contact layer, an absorbent component according to the first aspect of the invention, a transmission layer, a cover layer and an orifice to allow a negative pressure to be applied to the dressing.

The wound dressing or negative pressure apparatus 2100, which can alternatively be any wound dressing embodiment disclosed herein including without limitation wound dressing 100 or have any combination of features of any number of wound dressing embodiments disclosed herein, can be located over a wound site to be treated. The dressing 2100 forms a sealed cavity over the wound site.

When a wound packing material is used, once the wound dressing 2100 is sealed over the wound site, TNP is transmitted from a pump through the wound dressing 2100, through the wound packing material, and to the wound site. This negative pressure draws wound exudate and other fluids or secretions away from the wound site.

It is envisaged that the negative pressure range for the apparatus embodying the present disclosure may be between about -20 mmHg and -200 mmHg (note that these pressures are relative to normal ambient atmospheric pressure thus, -200 mmHg would be about 560 mmHg in practical terms). In one embodiment, the pressure range may be between about -40 mmHg and -150 mmHg. Alternatively a pressure range of up to -75 mmHg, up to -80 mmHg or over -80 mmHg can be used. Also in other embodiments a pressure range of below -75 mmHg could be used. Alternatively a pressure range of over-100 mmHg could be used or over-150 mmHg.

It will be appreciated that according to certain embodiments of the present disclosure, the pressure provided may be modulated over a period of time according to one or more desired and predefined pressure profiles. For example such a profile may include modulating the negative pressure between two predetermined negative pressures P1 and P2 such that pressure is held substantially constant at P1 for a pre-determined time period T1 and then adjusted by suitable means such as varying pump work or restricting fluid flow or the like, to a new predetermined pressure P2 where the pressure may be held substantially constant for a further predetermined time period T2. Two, three or four or more predetermined pressure values and respective time periods may be optionally utilized. Other embodiments may employ more complex amplitude/frequency wave forms of pressure flow profiles may also be provided e.g. sinusoidal, sore tooth, systolic-diastolic or the like.

As illustrated in Figures 5A-B a lower surface 2101 of the wound dressing 2100, which, again, can be any wound dressing embodiment disclosed herein including without limitation dressing embodiment 100 or have any combination of features of any number of wound dressing embodiments disclosed herein, can be provided by an optional wound contact layer 2102. The wound contact layer 2102 can be a polyurethane layer or polyethylene layer or other flexible layer which is perforated, for example via a hot pin process, laser ablation process, ultrasound process or in some other way or otherwise made permeable to liquid and gas. The wound contact layer has a lower surface 2101 and an upper surface 2103. The perforations 2104 are through holes in the wound contact layer which enables fluid to flow through the layer. The wound contact layer helps prevent tissue ingrowth into the other material of the wound dressing. The perforations are small enough to meet this requirement but still allow fluid through. For example, perforations formed as slits or holes having a size ranging from 0.025 mm to 1.2 mm are considered small enough to help prevent tissue ingrowth into the wound dressing while allowing wound exudate to flow into the dressing. The wound contact layer helps hold the whole wound dressing together and helps to create an air tight seal around the absorbent pad in order to maintain negative pressure at the wound. The wound contact layer also acts as a carrier for an optional lower and upper adhesive layer (not shown). For example, a lower pressure sensitive adhesive may be provided on the underside surface 2101 of the wound dressing whilst an upper pressure sensitive adhesive layer may be provided on the upper surface 2103 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized this helps adhere the wound dressing to the skin around a wound site.

A layer 2105 of porous material can be located above the wound contact layer. This porous layer, or transmission layer, 2105 allows transmission of fluid including liquid and gas away from a wound site into upper layers of the wound dressing. In particular, the transmission layer 2105 ensures that an open air channel can be maintained to communicate negative pressure over the wound area even when the absorbent component has absorbed substantial amounts of exudates. The layer should remain open under the typical pressures that will be applied during negative pressure wound therapy as described above, so that the whole wound site sees an equalized negative pressure. The layer 2105 is formed of a material having a three dimensional structure. For example, a knitted or woven spacer fabric (for example Baltex 7970 weft knitted polyester) or a nonwoven fabric could be used. Other materials could of course be utilized.

In some embodiments, the transmission layer comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 100 denier flat polyester and a third layer formed sandwiched between these two layers which is a region defined by a knitted polyester viscose, cellulose or the like monofilament fibre. Other materials and other linear mass densities of fibre could of course be used.

Whilst reference is made throughout this disclosure to a monofilament fibre it will be appreciated that a multistrand alternative could of course be utilized.

The top spacer fabric thus has more filaments in a yarn used to form it than the number of filaments making up the yarn used to form the bottom spacer fabric layer.

This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent component helps lock the liquid away or itself wicks the liquid onwards towards the cover layer where it can be transpired.

Preferably, to improve the liquid flow across the transmission layer (that is to say perpendicular to the channel region formed between the top and bottom spacer layers, the 3D fabric is treated with a dry cleaning agent (such as, but not limited to, perchloro ethylene) to help remove any manufacturing products such as mineral oils, fats and/or waxes used previously which might interfere with the hydrophilic capabilities of the transmission layer. In some embodiments, an additional manufacturing step can subsequently be carried in which the 3D spacer fabric is washed in a hydrophilic agent (such as, but not limited to, Feran Ice 30g/l available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

An absorbent component according to the invention 2110 is provided above the transmission layer 2105. The absorbent component forms a reservoir for fluid, particularly liquid, removed from the wound site and draws those fluids towards a cover layer 2140. With reference to Figures 5A and 5B, a masking or obscuring layer 2107 can be positioned beneath the cover layer 2140. In some embodiments, the masking layer 2107 can have any of the same features, materials, or other details of any of the other embodiments of the masking layers disclosed herein, including but not limited to having any viewing windows or holes. Additionally, the masking layer 2107 can be positioned adjacent to the cover layer, or can be positioned adjacent to any other dressing layer desired. In some embodiments, the masking layer 2107 can be adhered to or integrally formed with the cover layer. In some embodiments the masking layer 2107 may optionally contain a hole (not shown) directly adjacent to the port 2150 to improve air flow through the layer.

The absorbent component also prevents liquid collected in the wound dressing from flowing in a sloshing manner. The absorbent component 2110 also helps distribute fluid throughout the component via a wicking action so that fluid is drawn from the wound site and stored throughout the absorbent component. This helps prevent agglomeration in areas of the absorbent component. The capacity of the absorbent component must be sufficient to manage the exudates flow rate of a wound when negative pressure is applied. Since in use the absorbent component experiences negative pressures the superabsorbent material of the absorbent component is chosen to absorb liquid under such circumstances.

Preferably the absorbent component includes at least one through hole located so as to underlie the suction port. As illustrated in Figures 5A-B a single through hole can be used to produce an opening underlying the port 2150. It will be appreciated that multiple openings could alternatively be utilized. Additionally should more than one port be utilized according to certain embodiments of the present disclosure one or multiple openings may be made in the absorbent component in registration with each respective port. Although not essential to certain embodiments of the present disclosure the use of through holes in the absorbent component provides a fluid flow pathway which is particularly unhindered and this is useful in certain circumstances.

Where an opening is provided in the absorbent component the thickness of the component itself will act as a stand-off separating any overlying layer from the upper surface (that is to say the surface facing away from a wound in use) of the transmission layer 2105. An advantage of this is that the filter of the port is thus decoupled from the material of the transmission layer. This helps reduce the likelihood that the filter will be wetted out and thus will occlude and block further operation.

Use of one or more through holes in the absorbent component also has the advantage that during use if the absorbent component expands to absorb liquid it does not form a barrier through which further liquid movement and fluid movement in general cannot pass. In this way each opening in the absorbent component provides a fluid pathway between the transmission layer directly to the wound facing surface of the filter and then onwards into the interior of the port.

A gas impermeable, but moisture vapour permeable, cover layer 2140 can extend across the width of the wound dressing, which can be any wound dressing embodiment disclosed herein including without limitation dressing embodiment 100 or have any combination of features of any number of wound dressing embodiments disclosed herein. The cover layer, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way an effective chamber is made between the cover layer and a wound site where a negative pressure can be established. The cover layer 2140 is sealed to the wound contact layer 2102 in a border region 2200 around the circumference of the dressing, ensuring that no air is drawn in through the border area, for example via adhesive or welding techniques. The cover layer 140 protects the wound from external bacterial contamination (bacterial barrier) and allows liquid from wound exudates to be transferred through the layer and evaporated from the film outer surface. The cover layer 2140 typically comprises two layers; a polyurethane film and an adhesive pattern spread onto the film. The polyurethane film is moisture vapour permeable and may be manufactured from a material that has an increased water transmission rate when wet.

The absorbent component 2110 may be of a greater area than the transmission layer 2105, such that the absorbent component overlaps the edges of the transmission layer 2105, thereby ensuring that the transmission layer does not contact the cover layer 2140. This provides an outer channel 2115 of the absorbent component 2110 that is in direct contact with the wound contact layer 2102, which aids more rapid absorption of exudates to the absorbent component. Furthermore, this outer channel 2115 ensures that no liquid is able to pool around the circumference of the wound cavity, which may otherwise seep through the seal around the perimeter of the dressing leading to the formation of leaks.

In order to ensure that the air channel remains open when a vacuum is applied to the wound cavity, the transmission layer 2105 must be sufficiently strong and non-compliant to resist the force due to the pressure differential. However, if this layer comes into contact with the relatively delicate cover layer 2140, it can cause the formation of pin-hole openings in the cover layer 2140 which allow air to leak into the wound cavity. This may be a particular problem when a switchable type polyurethane film is used that becomes weaker when wet. The absorbent component 2110 is generally formed of a relatively soft, non-abrasive material compared to the material of the transmission layer 2105 and therefore does not cause the formation of pin-hole openings in the cover layer. Thus by providing an absorbent component 2110 that is of greater area than the transmission layer 2105 and that overlaps the edges of the transmission layer 2105, contact between the transmission layer and the cover layer is prevented, avoiding the formation of pin-hole openings in the cover layer 2140.

The absorbent component 2110 is positioned in fluid contact with the cover layer 2140. As the absorbent component absorbs wound exudate, the exudate is drawn towards the cover layer 2140, bringing the water component of the exudate into contact with the moisture vapour permeable cover layer. This water component is drawn into the cover layer itself and then evaporates from the top surface of the dressing. In this way, the water content of the wound exudate can be transpired from the dressing, reducing the volume of the remaining wound exudate that is to be absorbed by the absorbent component 2110, and increasing the time before the dressing becomes full and must be changed. This process of transpiration occurs even when negative pressure has been applied to the wound cavity, and it has been found that the pressure difference across the cover layer when a negative pressure is applied to the wound cavity has negligible impact on the moisture vapour transmission rate across the cover layer.

An orifice 2145 is provided in the cover film 2140 to allow a negative pressure to be applied to the dressing 2100. A suction port 2150 is sealed to the top of the cover film 2140 over the orifice 2145, and communicates negative pressure through the orifice 2145. A length of tubing 2220 may be coupled at a first end to the suction port 2150 and at a second end to a pump unit (not shown) to allow fluids to be pumped out of the dressing. The port may be adhered and sealed to the cover film 2140 using an adhesive such as an acrylic, cyanoacrylate, epoxy, UV curable or hot melt adhesive. The port 2150 is formed from a soft polymer, for example a polyethylene, a polyvinyl chloride, a silicone or polyurethane having a hardness of 30 to 90 on the Shore A scale.

An aperture or through-hole 2146 is provided in the absorbent component 2110 beneath the orifice 2145 such that the orifice is connected directly to the transmission layer 2105. This allows the negative pressure applied to the port 2150 to be communicated to the transmission layer 2105 without passing through the absorbent component 2110. This ensures that the negative pressure applied to the wound site is not inhibited by the absorbent component as it absorbs wound exudates. In other embodiments, no aperture may be provided in the absorbent component 2110, or alternatively a plurality of apertures underlying the orifice 2145 may be provided.

As shown in Figure 5A, one embodiment of the wound dressing 2100 comprises an aperture 2146 in the absorbent component 2110 situated underneath the port 2150. In use, for example when negative pressure is applied to the dressing 2100, a wound facing portion of the port 150 may thus come into contact with the transmission layer 2105, which can thus aid in transmitting negative pressure to the wound site even when the absorbent component 2110 is filled with wound fluids. Some embodiments may have the cover layer 2140 be at least partly adhered to the transmission layer 2105. In some embodiments, the aperture 2146 is at least 1-2 mm larger than the diameter of the wound facing portion of the port 2150, or the orifice 2145.

A filter element 2130 that is impermeable to liquids, but permeable to gases is provided to act as a liquid barrier, and to ensure that no liquids are able to escape from the wound dressing. The filter element may also function as a bacterial barrier. Typically the pore size is 0.2µm. Suitable materials for the filter material of the filter element 2130 include 0.2 micron Gore^{™} expanded PTFE from the MMT range, PALL Versapore^{™} 200R, and Donaldson^{™} TX6628. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter. The filter element can be attached or sealed to the port and/or the cover film 2140 over the orifice 2145. For example, the filter element 2130 may be moulded into the port 2150, or may be adhered to both the top of the cover layer 2140 and bottom of the port 2150 using an adhesive such as, but not limited to, a UV cured adhesive.

It will be understood that other types of material could be used for the filter element 2130. More generally a microporous membrane can be used which is a thin, flat sheet of polymeric material, this contains billions of microscopic pores. Depending upon the membrane chosen these pores can range in size from 0.01 to more than 10 micrometres. Microporous membranes are available in both hydrophilic (water filtering) and hydrophobic (water repellent) forms. In some embodiments of the disclosure, filter element 2130 comprises a support layer and an acrylic copolymer membrane formed on the support layer. Preferably the wound dressing 2100 according to certain embodiments of the present disclosure uses microporous hydrophobic membranes (MHMs). Numerous polymers may be employed to form MHMs. For example, PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing airto flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter element 2130 may also include an odour absorbent material, for example activated charcoal, carbon fibre cloth or Vitec Carbotec-RT Q2003073 foam, or the like. For example, an odour absorbent material may form a layer of the filter element 2130 or may be sandwiched between microporous hydrophobic membranes within the filter element.

The filter element 2130 thus enables gas to be exhausted through the orifice 2145. Liquid, particulates and pathogens however are contained in the dressing.

In Figure 5B, an embodiment of the wound dressing 2100 is illustrated which comprises spacer elements 2152, 2153 in conjunction with the port 2150 and the filter 2130. With the addition of such spacer elements 2152, 2153, the port 2150 and filter 2130 may be supported out of direct contact with the absorbent component 2110 and/or the transmission layer 2105. The absorbent component 2110 may also act as an additional spacer element to keep the filter 2130 from contacting the transmission layer 2105. Accordingly, with such a configuration contact of the filter 2130 with the transmission layer 2105 and wound fluids during use may thus be minimized. As contrasted with the embodiment illustrated in Figure 5A, the aperture 2146 through the absorbent component 2110 may not necessarily need to be as large or larger than the port 2150, and would thus only need to be large enough such that an air path can be maintained from the port to the transmission layer 2105 when the absorbent component 2110 is saturated with wound fluids.

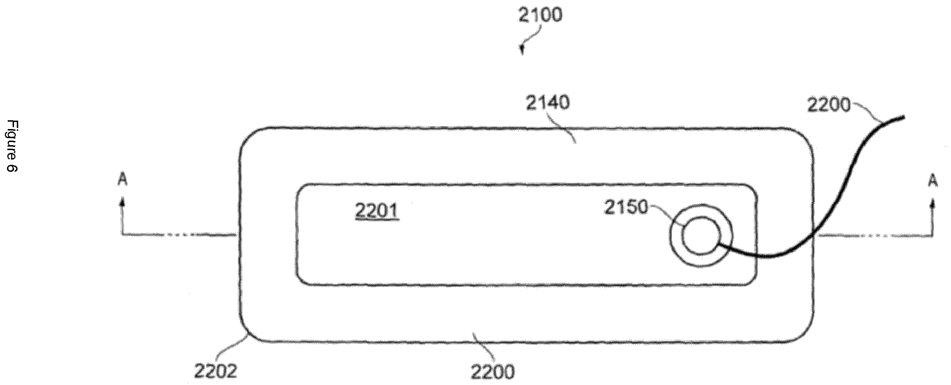

In particular for embodiments with a single port 2150 and through hole, it may be preferable for the port 2150 and through hole to be located in an off-centre position as illustrated in Figures 5A-B and in Figure 6. Such a location may permit the dressing 2100 to be positioned onto a patient such that the port 2150 is raised in relation to the remainder of the dressing 2100. So positioned, the port 2150 and the filter 2130 may be less likely to come into contact with wound fluids that could prematurely occlude the filter 2130 so as to impair the transmission of negative pressure to the wound site.

The wound dressing 2100 and its methods of manufacture and use as described herein may also incorporate features, configurations and materials described in the following patents and patent applications that are all incorporated by reference in their entireties herein: U.S. Patent Nos. 7,524,315, 7,708,724, and 7,909,805; U.S. Patent Application Publication Nos. 2005/0261642, 2007/0167926, 2009/0012483, 2009/0254054, 2010/0160879, 2010/0160880, 2010/0174251, 2010/0274207, 2010/0298793, 2011/0009838, 2011/0028918, 2011/0054421, and 2011/0054423; as well as U.S. App. Serial. Nos. 12/941,390, filed November 8, 2010, 29/389,782, filed April 15, 2011, and 29/389,783, filed April 15, 2011. From these incorporated by reference patents and patent applications, features, configurations, materials and methods of manufacture or use for similar components to those described in the present disclosure may be substituted, added or implemented into embodiments of the present application.

In operation the wound dressing 2100 is sealed over a wound site forming a wound cavity. A pump unit applies a negative pressure at a connection portion 2154 of the port 2150 which is communicated through the orifice 2145 to the transmission layer 2105. Fluid is drawn towards the orifice through the wound dressing from a wound site below the wound contact layer 2102. The fluid moves towards the orifice through the transmission layer 2105. As the fluid is drawn through the transmission layer 2105 wound exudate is absorbed into the absorbent component 2110.

Turning to Figure 6 which illustrates a wound dressing 2100 in accordance with an embodiment of the present disclosure one can see the upper surface of the cover layer 2140 which extends outwardly away from a centre of the dressing into a border region 2200 surrounding a central raised region 2201 overlying the transmission layer 2105 and the absorbent component 2110. As indicated in Figure 6 the general shape of the wound dressing is rectangular with rounded corner regions 2202. It will be appreciated that wound dressings according to other embodiments of the present disclosure can be shaped differently such as square, circular or elliptical dressings, or the like.

The wound dressing 2100 may be sized as necessary for the size and type of wound it will be used in. In some embodiments, the wound dressing 2100 may measure between 20 and 40 cm on its long axis, and between 10 to 25 cm on its short axis. For example, dressings may be provided in sizes of 10 × 20 cm, 10 × 30 cm, 10 × 40 cm, 15 × 20 cm, and 15 × 30 cm. In some embodiments, the wound dressing 2100 may be a square-shaped dressing with sides measuring between 15 and 25 cm (e.g., 15 × 15 cm, 20 × 20 cm and 25 × 25 cm). The absorbent component 2110 may have a smaller area than the overall dressing, and in some embodiments may have a length and width that are both about 3 to 10 cm shorter, more preferably about 5 cm shorter, than that of the overall dressing 2100. In some rectangular-shape embodiments, the absorbent component 2110 may measure between 10 and 35 cm on its long axis, and between 5 and 10 cm on its short axis. For example, absorbent components may be provided in sizes of 5.6 × 15 cm or 5 × 10 cm (for 10 × 20 cm dressings), 5.6 × 25 cm or 5 × 20 cm (for 10 × 30 cm dressings), 5.6 × 35 cm or 5 × 30 cm (for 10 × 40 cm dressings), 10 × 15 cm (for 15 × 20 cm dressings), and 10 × 25 cm (for 15 × 30 cm dressings). In some square-shape embodiments, the absorbent component 2110 may have sides that are between 10 and 20 cm in length (e.g., 10 × 10 cm for a 15 × 15 cm dressing, 15 × 15 cm for a 20 × 20 cm dressing, or 20 × 20 cm for a 25 × 25 cm dressing). The transmission layer 2105 is preferably smaller than the absorbent component, and in some embodiments may have a length and width that are both about 0.5 to 2 cm shorter, more preferably about 1 cm shorter, than that of the absorbent component. In some rectangular-shape embodiments, the transmission layer may measure between 9 and 34 cm on its long axis and between 3 and 5 cm on its short axis. For example, transmission layers may be provided in sizes of 4.6 × 14 cm or 4 × 9 cm (for 10 × 20 cm dressings), 4.6 × 24 cm or 4 × 19 cm (for 10 × 30 cm dressings), 4.6 × 34 cm or 4 × 29 cm (for 10 × 40 cm dressings), 9 × 14 cm (for 15 × 20 cm dressings), and 9 × 24 cm (for 15 × 30 cm dressings). In some square-shape embodiments, the transmission layer may have sides that are between 9 and 19 cm in length (e.g., 9 × 9 cm for a 15 × 15 cm dressing, 14 × 14 cm for a 20 × 20 cm dressing, or 19 × 19 cm for a 25 × 25 cm dressing).

It will be understood that according to embodiments of the present disclosure the wound contact layer is optional. This layer is, if used, porous to water and faces an underlying wound site. A transmission layer 2105 such as an open celled foam, or a knitted or woven spacer fabric is used to distribute gas and fluid removal such that all areas of a wound are subjected to equal pressure. The cover layer together with the filter layer forms a substantially liquid tight seal over the wound. Thus when a negative pressure is applied to the port 2150 the negative pressure is communicated to the wound cavity below the cover layer. This negative pressure is thus experienced at the target wound site. Fluid including air and wound exudate is drawn through the wound contact layer and transmission layer 2105. The wound exudate drawn through the lower layers of the wound dressing is dissipated and absorbed into the absorbent component 2110 where it is collected and stored. Air and moisture vapour is drawn upwards through the wound dressing through the filter layer and out of the dressing through the suction port. A portion of the water content of the wound exudate is drawn through the absorbent component and into the cover layer 2140 and then evaporates from the surface of the dressing.

As discussed above, when a negative pressure is applied to a wound dressing sealed over a wound site, in some dressing embodiments disclosed herein, fluids including wound exudate are drawn from the wound site and through the transmission layer 2105 toward the orifice 2145. Wound exudate is then drawn into the absorbent component 2110 where it is absorbed. However, some wound exudate may not be absorbed and may move to the orifice 2145. Filter element 2130 provides a barrier that stops any liquid in the wound exudate from entering the connection portion 2154 of the suction port 2150. Therefore, unabsorbed wound exudate may collect underneath the filter element 2130. If sufficient wound exudate collects at the filter element, a layer of liquid will form across the surface of filter element 2130 and the filter element will become blocked as the liquid cannot pass through the filter element 2130 and gases will be stopped from reaching the filter element by the liquid layer. Once the filter element becomes blocked, negative pressure can no longer be communicated to the wound site, and the wound dressing must be changed for a fresh dressing, even though the total capacity of the absorbent component has not been reached.

In a preferred embodiment, the port 2150, along with any aperture 2146 in the absorbing layer 2110 situated below it, generally aligns with the mid-longitudinal axis A-A illustrated in Figure 6. Preferably, the port 2150 and any such aperture 2146 are situated closer to one end of the dressing, contrasted with a central position. In some embodiments, the port may be located at a corner of the dressing 2100, which again can be any dressing embodiment disclosed herein including without limitation dressing embodiment 100. For example, in some rectangular embodiments, the port 2150 may be located between 4 and 6 cm from the edge of the dressing, with the aperture 146 located 2 to 3 cm from the edge of the absorbent component. In some square embodiments, the port 2150 may be located between 5 to 8 cm from the corner of the dressing, with the aperture 2146 located 3 to 5 cm from the corner of the absorbent component.

Certain orientations of the wound dressing may increase the likelihood of the filter element 130 becoming blocked in this way, as the movement of the wound exudate through the transmission layer may be aided by the effect of gravity. Thus, if due to the orientation of the wound site and wound dressing, gravity acts to increase the rate at which wound exudate is drawn towards the orifice 2145, the filter may become blocked with wound exudate more quickly. Thus, the wound dressing would have to be changed more frequently and before the absorbent capacity of the absorbent component 2110 has been reached.

In order to avoid the premature blocking of the wound dressing 2100 by wound exudate drawn towards the orifice 2145 some embodiments of the disclosure include at least one element configured to reduce the rate at which wound exudate moves towards the orifice 2145. The at least one element may increase the amount of exudate that is absorbed into the absorbent component before reaching the orifice 2145 and/or may force the wound exudate to follow a longer path through the dressing before reaching the orifice 2145, thereby increasing the time before the wound dressing becomes blocked.

As still further options the dressing can contain anti-microbial e.g. nanocrystalline silver agents on the wound contact layer and/or silver sulphur diazine in the absorbent component. These may be used separately or together. These respectively kill micro-organisms in the wound and micro-organisms in the absorption matrix. As a still further option other active components, for example, pain suppressants, such as ibuprofen, may be included. Also agents which enhance cell activity, such as growth factors or that inhibit enzymes, such as matrix metalloproteinase inhibitors, such as tissue inhibitors of metalloproteinase (TIMPS) or zinc chelators could be utilized. As a still further option odour trapping elements such as activated carbon, cyclodextrine, zeolite or the like may be included in the absorbent component or as a still further layer above the filter layer.

Whilst certain embodiments of the present disclosure have so far been described in which the transmission layer is formed as a 3D knit layer, e.g., two layers spaced apart by a monofilament layer, it will be appreciated that certain embodiments of the present disclosure are not restricted to the use of such a material. In some embodiments, as an alternative to such a 3D knit material one or more layers of a wide variety of materials could be utilized. In each case, according to embodiments of the present disclosure, the openings presented by layers of the transmission layer are wider and wider as one moves away from the side of the dressing which, in use will be located proximate to the wound. In some embodiments, the transmission layer may be provided by multiple layers of open celled foam. In some embodiments, the foam is reticulated open cell foam. Preferably, the foam is hydrophilic or able to wick aqueous based fluids. The pore size in each layer is selected so that in the foam layer most proximate to the wound side in use the pores have a smallest size. If only one further foam layer is utilized that includes pore sizes which are greater than the pore sizes of the first layer. This helps avoid solid particulate being trapped in the lower layer which thus helps maintain the lower layer in an open configuration in which it is thus able to transmit air throughout the dressing. In certain embodiments, two, three, four or more foam layers may be included. The foam layers may be integrally formed, for example, by selecting a foam having a large pore size and then repeatedly dipping this to a lesser and lesser extent into material which will clog the pores or alternatively, the transmission layer formed by the multiple foam layers may be provided by laminating different types of foam in a layered arrangement or by securing such layers of foam in place in a known manner.

According to certain embodiments of the present disclosure, the transmission layer is formed by multiple layers of mesh instead of foam or 3D knit materials. For example, fine gauze mesh may be utilized for a wound facing side of the transmission layer and a Hessian mesh having a larger pore size may be located on a distal side of the gauze mesh facing away from the wound in use. The one, two, three or more layers of mesh can be secured together in an appropriate manner, such as being stitched or adhered together or the like. The resultant mat of fibres provides a transmittal layer through which air can be transmitted in the dressing but by selecting the opening sizes in the meshes as one moves through the dressing away from the wound contact side, the accumulation of solid particulate matter in lower layers can be avoided.

With the embodiments of the present disclosure, a wound dressing is provided that helps improve patient concordance with instructions for use, helps improve patients" quality of life, and also helps a clinician observe and monitor a patient's wound.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Throughout the description and claims of this specification, the singular encompasses the plural unless the context otherwise requires. In particular, where the indefinite article is used, the specification is to be understood as contemplating plurality as well as singularity, unless the context requires otherwise.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," "essentially" and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

## Claims

1. An absorbent component for a wound dressing comprising a stitch bonded nonwoven substrate
wherein the nonwoven substrate is stitch bonded with an elastomeric yarn and
wherein the nonwoven substrate comprises a blend of superabsorbent material and/or a gelling material and non-superabsorbent and non-gelling support material(s).

2. An absorbent component according to claim 1 wherein the elastomeric yarn comprises at least 50%, at least 60%, at least 70%, at least 80% or at least 90% elastomeric material.

3. An absorbent component according to claim1 or claim 2 wherein the elastomeric material is spandex.

4. An absorbent component according to claim 1 wherein the nonwoven substrate comprises at least 30%, at least 40%, at least 50%, at least 60%, at least 70%, at least 80%, or at least 90% superabsorbent and/or gelling material.

5. An absorbent component according to any one of the preceding claims wherein the nonwoven substrate comprises a superabsorbent material.

6. An absorbent component according to claim 5 wherein the superabsorbent material comprises a polysaccharide or modified polysaccharide.

7. An absorbent component according to any one of the preceding claims wherein the nonwoven substrate is a carded nonwoven substrate.

8. A wound dressing comprising an absorbent component according to any one of claims 1 to 7.

9. A negative pressure apparatus comprising a wound contact layer, an absorbent component according to any one of claims 1 to 7, a transmission layer, a cover layer and an orifice for applying reduced pressure.

## Patentansprüche

1. Eine absorbierende Komponente für einen Wundverband, beinhaltend ein vernähtes Vliessubstrat,
wobei das Vliessubstrat mit einem elastomeren Garn vernäht ist und
wobei das Vliessubstrat ein Gemisch aus superabsorbierendem Material und/oder
einem gelierenden Material und nicht superabsorbierenden und nicht gelierenden Trägermaterial(ien) beinhaltet.

2. Absorbierende Komponente gemäß Anspruch 1, wobei das elastomere Garn mindestens 50 %, mindestens 60 %, mindestens 70 %, mindestens 80 % oder mindestens 90 % elastomeres Material beinhaltet.

3. Absorbierende Komponente gemäß Anspruch 1 oder Anspruch 2, wobei das elastomere Material Spandex ist.

4. Absorbierende Komponente gemäß Anspruch 1, wobei das Vliessubstrat mindestens 30 %, mindestens 40 %, mindestens 50 %, mindestens 60 %, mindestens 70 %, mindestens 80 % oder mindestens 90 % superabsorbierendes und/oder gelierendes Material beinhaltet.

5. Absorbierende Komponente gemäß einem der vorhergehenden Ansprüche, wobei das Vliessubstrat ein superabsorbierendes Material beinhaltet.

6. Absorbierende Komponente gemäß Anspruch 5, wobei das superabsorbierende Material ein Polysaccharid oder modifiziertes Polysaccharid beinhaltet.

7. Absorbierende Komponente gemäß einem der vorhergehenden Ansprüche, wobei das Vliessubstrat ein kardiertes Vliessubstrat ist.

8. Wundverband, beinhaltend eine absorbierende Komponente gemäß einem der Ansprüche 1 bis 7.

9. Eine Unterdruckeinrichtung, beinhaltend eine Wundkontaktschicht, eine absorbierende Komponente gemäß einem der Ansprüche 1 bis 7, eine Übertragungsschicht, eine Deckschicht und eine Öffnung zum Anlegen von reduziertem Druck.

## Revendications

1. Un composant absorbant destiné à un pansement pour plaie comprenant un substrat non-tissé lié par couture
dans lequel le substrat non-tissé est lié par couture à l'aide d'un fil élastomère et
dans lequel le substrat non-tissé comprend un mélange homogène de matériau superabsorbant et/ou d'un matériau gélifiant et de matériau(x) de support non superabsorbant et non gélifiant.

2. Un composant absorbant selon la revendication 1 dans lequel le fil élastomère comprend au moins 50 %, au moins 60 %, au moins 70 %, au moins 80 % ou au moins 90 % de matériau élastomère.

3. Un composant absorbant selon la revendication 1 ou la revendication 2 dans lequel le matériau élastomère est l'élasthanne.

4. Un composant absorbant selon la revendication 1 dans lequel le substrat non-tissé comprend au moins 30 %, au moins 40 %, au moins 50 %, au moins 60 %, au moins 70 %, au moins 80 %, ou au moins 90 % de matériau superabsorbant et/ou gélifiant.

5. Un composant absorbant selon n'importe laquelle des revendications précédentes dans lequel le substrat non-tissé comprend un matériau superabsorbant.

6. Un composant absorbant selon la revendication 5 dans lequel le matériau superabsorbant comprend un polysaccharide ou un polysaccharide modifié.

7. Un composant absorbant selon n'importe laquelle des revendications précédentes dans lequel le substrat non-tissé est un substrat non-tissé cardé.

8. Un pansement pour plaie comprenant un composant absorbant selon n'importe laquelle des revendications 1 à 7.

9. Un appareil à pression négative comprenant une couche en contact avec la plaie, un composant absorbant selon n'importe laquelle des revendications 1 à 7, une couche de transmission, une couche de recouvrement et un orifice pour l'application de pression réduite.
